# EUROPEAN PATENT APPLICATION

(11) **EP 4 176 831 A1**
(43) Date of publication of application: **10.05.2023**
(21) Application number: 21206825.8
(22) Date of filing: 07.11.2021
(51) Int. Cl.: A61B 17/32, A61B 17/00, A61B 90/00

(54) **MEDICAL INSTRUMENT, IN PARTICULAR FOR PERCUTANEOUS SURGICAL/MEDICAL PROCEDURES**

(71) Applicant: SPIRECUT SA, 1700 Fribourg (CH)
(72) Inventor: MOUNGONDO, Fabian, 7340 Colfontaine (BE); SCHUIND, Frédéric, 1801 Le Mont-Pèlerin (CH)
(74) Representative: Noll, Ronald

(57) **Abstract**

There is in particular described a medical instrument (10), in particular for percutaneous surgical/medical procedures, comprising a handle portion (H) designed to allow handling, orientation and manipulation of the medical instrument (10) by an operator and an elongated rod member (100) secured to the handle portion (H). The elongated rod member (100) extends from the handle portion (H) up to a distal end and is preferably curved and/or bent substantially within a defined plane (P0). At least one portion of the elongated rod member (100), proximate to the distal end, is provided with a marking (200) distinguishable under sonography. The marking (200) is designed as a rotationally asymmetric marking (200A, 200B) configured to allow a location and a rotational orientation of the elongated rod member (100) to be uniquely detectable in sonographic imagery.

## Description

### TECHNICAL FIELD

The present invention generally relates to a medical instrument as used, in particular, for carrying out percutaneous surgical/medical procedures, especially percutaneous release procedures on upper or lower limbs, such as but not limited to percutaneous carpal tunnel release and percutaneous A1 pulley release. The invention is also applicable to other surgical or medical procedures that are performed under sonography such as but not limited to biopsies, infiltrations, injections, needling, removal of foreign bodies, tenolysis, tenotomy, tenotomy-lengthening, aponeurotomy, neurolysis, neurotomy and any other surgical release procedure beyond carpal tunnel release and digital/thumb pulley release, such as for the treatment of Dupuytren disease, de Quervain syndrome, tennis elbow, Morton neuroma, and in general in any percutaneous procedure at any anatomical location.

### BACKGROUND OF THE INVENTION

Carpal tunnel syndrome (CTS) and trigger finger syndrome (TFS) can conveniently be treated by percutaneous surgical release procedures. These procedures are typically carried out using simple puncture needles or more complex hook knives or push knives. Such medical instruments may also be used for other surgical release procedures on upper or lower limbs, such as for the treatment of Morton's neuroma, tarsal tunnel release, de Quervain syndrome, epicondylalgy, shoulder surgery, and similar release procedures, which list is not meant to be exhaustive.

Various solutions are known in the art, for instance from US Patents Nos. US 5,507,800 A, US 5,029,573 A, and US 8,603,124 B1, but these solutions are not suitable for carrying out percutaneous release procedures.

Percutaneous release procedures can be carried out using simple puncture needles. This solution is not satisfying however since the release procedure is not easy to carry out and potentially dangerous for adjacent structures. A more elaborated solution is disclosed in US Patent No. US 5,782,850 A but the configuration of the relevant medical instrument disclosed therein is still relatively complex and may cause unwanted damage to surrounding tissues and structures, especially during withdrawal of the medical instrument.

International (PCT) Publication No. WO 2020/003263 A1 in the name of the present Applicant, the content of which is incorporated herein by reference in its entirety, discloses various medical instruments for truly percutaneous release procedures, especially such medical instruments that are designed to be used under sonography, i.e. in combination with a sonography probe. According to WO 2020/003263 A1, it is especially contemplated to provide a plurality of markings in the form of embossings along different portions of the elongated rod member of the medical instrument, which markings are designed to be distinguishable under sonography. While the provision of embossings may help identifying the elongated rod member in sonographic imagery and detect a position of the distal end of the elongated rod member, such markings as specifically contemplated in WO 2020/003263 A1 may not necessarily be sufficient to properly determine the orientation of the elongated rod member and of its tip.

French Patent Publication No. FR 2 272 633 A1 discloses a puncture needle of the type comprising peripheral annular grooves that are distributed along a length of the puncture needle, which puncture needle is in essence rectilinear. These peripheral annular grooves have the effect of dispersing ultrasonic waves and therefore facilitate location of the puncture needle in sonographic imagery. Further solutions aimed at enhancing echogenicity of medical instruments are disclosed in US Patents Nos. US 4,582,061 A, US 4,977, 897 A, US 5,490,521 A and European Patents Nos. EP 1 132 049 B1, EP 2 384 146 B1.

While these solutions provide some ability to enhance echogenicity, they are not entirely satisfactory in that they do not allow to detect the actual rotational orientation of the medical instrument about its axis. There therefore remains a need for an improved solution that enables not only the location but also the rotational orientation of the relevant elongated rod member of the medical instrument to be detected and identified.

Referring to the medical instruments disclosed in WO 2020/003263 A1, there also remains a need to provide such a medical instrument which has a better ability to cut through tissue, especially ligaments which are reasonably hard tissues to cut through, while also improving echogenicity of the cutting end of the medical instrument.

### SUMMARY OF THE INVENTION

A general aim of the invention is to provide an improved medical instrument, in particular for (but not limited to) percutaneous release procedures, especially percutaneous release procedures on upper or lower limbs, such as percutaneous carpal tunnel release or percutaneous digital/thumb pulley release, as well as other percutaneous release procedures used in the treatment of the conditions or syndromes as for instance listed in the introductory part of this description.

More specifically, an aim of the present invention is to improve echogenicity of such medical instruments that especially comprise an elongated rod member extending from a handle portion up to a distal end and being preferably curved and/or bent substantially within a defined plane with a view to facilitate identification and detection of a location as well as of a rotational orientation of the elongated rod member and of its distal end especially.

These aims, and others, are achieved, in accordance with a first aspect of the invention, thanks to the solution defined in claim 1, namely a medical instrument, in particular for percutaneous surgical/medical procedures, comprising a handle portion designed to allow handling, orientation and manipulation of the medical instrument by an operator and an elongated rod member secured to the handle portion, which elongated rod member extends from the handle portion up to a distal end and is preferably curved and/or bent substantially within a defined plane. At least one portion of the elongated rod member, proximate to the distal end, is provided with a marking distinguishable under sonography. According to this first aspect of the invention, the marking is designed as a rotationally asymmetric marking configured to allow a location and a rotational orientation of the elongated rod member to be uniquely detectable in sonographic imagery.

In accordance with a particularly preferred embodiment, the marking includes at least one rotationally asymmetric groove formed along a periphery of the elongated rod member.

In the context of this particularly preferred embodiment, the marking may in particular include first and second rotationally asymmetric grooves extending in opposite directions along the elongated rod member.

Advantageously, each rotationally asymmetric groove may consist of one or more helical turns. Even more specifically, in the context of the aforementioned embodiment having first and second rotationally asymmetric grooves extending in opposite directions along the elongated rod member, a distance separating a proximal end of the first rotationally asymmetric groove and a proximal end of the second rotationally asymmetric groove is substantially equal to a pitch of each rotationally asymmetric groove. Said distance and said pitch are preferably of approximately 2 mm.

In accordance with a further advantageous embodiment, each rotationally asymmetric groove may exhibit a width comprised between 0.5 mm and 1 mm and/or a depth comprised between 0.1 mm and 0.2 mm.

Furthermore, in one embodiment variant, a depth of each rotationally asymmetric groove may be constant. In another embodiment variant, a depth of each rotationally asymmetric groove may vary along a length of the rotationally asymmetric groove, which other embodiment variant is particularly advantageous in that a further variable enhancing and modulating echogenicity is introduced.

As a further refinement, one may additionally contemplate that an inner surface of each rotationally asymmetric groove be structured to further enhance echogenicity.

In accordance with another embodiment of the invention, the distal end of the elongated rod member preferably comprises a tapered end configured to act as a cutting device to sever tissue, the tapered end being at least partly delineated by first and second opposite surfaces, namely a first, bevelled surface that is inclined with respect to the defined plane and a second, recessed surface that is substantially parallel to the defined plane. In effect, this latter aspect forms another aspect of the invention that is potentially applicable independently of the aforementioned first aspect of the invention.

In other words, in accordance with a second aspect of the invention, as defined in independent claim 10, there is further provided a medical instrument for percutaneous release procedures, comprising a handle portion designed to allow handling, orientation and manipulation of the medical instrument by an operator and an elongated rod member secured to the handle portion, which elongated rod member extends from the handle portion up to a distal end and is curved and/or bent substantially within a defined plane. The distal end of the elongated rod member comprises a tapered end configured to act as a cutting device to sever tissue. According to this second aspect of the invention, the tapered end of the elongated rod member is at least partly delineated by first and second opposite surfaces, namely a first, bevelled surface that is inclined with respect to the defined plane and a second, recessed surface that is substantially parallel to the defined plane.

This particular configuration of the tapered end of the elongated rod member is especially aimed at enhancing echogenicity of the distal end of the medical instrument, and additional measures are contemplated hereafter with a view to further enhance echogenicity of the distal end of the medical instrument.

By way of preference, the tapered end of the elongated rod member may be further delineated by a pair of side surfaces forming side bevels alongside at least a portion of the bevelled surface and of the recessed surface. In this context, an angle of inclination of each side surface with respect to the recessed surface is preferably comprised between 25° and 35°.

Furthermore, an angle of inclination of a plane comprising the bevelled surface with respect to the defined plane (and thus with respect to the recessed surface) is comprised between 10° and 15°.

In accordance with an advantageous embodiment, a thickness of the tapered end, as measured perpendicularly to the defined plane, does not exceed 1 mm, and/or a width of the tapered end, as measured within the defined plane, does not exceed 2 mm.

In accordance with an advantageous variant of the invention, the tapered end includes a longitudinal ridge that projects away from the recessed surface. The presence of this longitudinal ridge is helpful in that echogenicity of the tapered end is improved as a result. By way of preference, a height of the longitudinal ridge, as measured perpendicularly to the defined plane, does not exceed 0.5 mm. Furthermore, a leading edge of the longitudinal ridge may be tapered and may be stopping short of a leading edge of the tapered end with a view to further minimize interference during insertion of the medical instrument in the area to be treated and prevent any impact on the cutting properties and sharpness of the leading edge of the tapered end.

Preferably, the elongated rod member extends along a generatrix and a lateral breadth of the elongated rod member as measured at any point along the generatrix, up to an including the distal end of the elongated rod member, does not exceed 2 mm.

Furthermore, a cross-sectional area of the elongated rod member preferably does not exceed 2.5 mm².

In addition, the elongated rod member may especially have a substantially circular or polygonal cross-section, upstream of the distal end of the elongated rod member, whose lateral breadth does not exceed 1.5 mm.

Further advantageous embodiments of the invention are discussed below.

### BRIEF DESCRIPTION OF THE DRAWINGS

Other features and advantages of the present invention will appear more clearly from reading the following detailed description of embodiments of the invention which are presented solely by way of non-restrictive examples and illustrated by the attached drawings in which:
Figure 1A is a perspective view of a medical instrument in accordance with a first embodiment of the invention, which medical instrument is particularly suited for percutaneous carpal tunnel release in the treatment of the carpal tunnel syndrome;
Figure 1B is a perspective view of the medical instrument of Figure 1A shown from a different viewing angle;
Figures 1C and 1D are enlarged views of the distal end (or tip) of the elongated rod member of the medical instrument shown in Figures 1A and 1B, respectively;
Figure 2A is a side view of an elongated rod member of a medical instrument in accordance with a variant of the first embodiment shown in Figures 1A-D, as shown perpendicularly to the defined plane in which the elongated rod member extends;
Figure 2B is an enlarged view of the distal end of the elongated rod member of Figure 2A showing a tapered end thereof in greater detail;
Figure 2C is an enlarged side view of the tapered end of the elongated rod member of Figures 2A-B;
Figure 2D is a cross-sectional view of the tapered end shown in Figures 2B-C as taken along sectional plane A-A identified in Figure 2B;
Figure 2E is an enlarged, partially cut-out view of part of the marking provided along a portion of the elongated rod member of Figure 2A, proximate to the distal end thereof
Figure 3A is an enlarged side view of the tapered end of the elongated rod member of a medical instrument in accordance with a further variant of the embodiments shown in Figures 1A-D and 2A-E;
Figure 3B is a cross-sectional view of the tapered end shown in Figures 3A as taken along a same sectional plane as in Figure 2D;
Figure 4 is an enlarged side view of the tapered end of the elongated rod member of a medical instrument in accordance with a variant of the embodiment shown in Figures 3A-B;
Figure 5A is a perspective view of a medical instrument in accordance with a second embodiment of the invention, which medical instrument is particularly suited for percutaneous A1 pulley release in the treatment of the trigger finger syndrome;
Figure 5B is a perspective view of the medical instrument of Figure 5A shown from a different viewing angle;
Figures 5C and 5D are enlarged views of the distal end (or tip) of the elongated rod member of the medical instrument shown in Figures 5A and 5B, respectively;
Figure 6A is a side view of an elongated rod member of a medical instrument in accordance with a variant of the second embodiment shown in Figures 5A-D, as shown perpendicularly to the defined plane in which the elongated rod member extends;
Figure 6B is an enlarged view of the distal end of the elongated rod member of Figure 6A showing a tapered end thereof in greater detail;
Figure 6C is an enlarged side view of the tapered end of the elongated rod member of Figures 6A-B;
Figure 6D is a cross-sectional view of the tapered end shown in Figures 6B-C as taken along sectional plane B-B identified in Figure 6B;
Figure 6E is an enlarged view of part of the marking provided along a portion of the elongated rod member of Figure 6A, proximate to the distal end thereof;
Figure 7A is an enlarged side view of the tapered end of the elongated rod member of a medical instrument in accordance with a further variant of the embodiments shown in Figures 5A-D and 6A-E;
Figure 7B is a cross-sectional view of the tapered end shown in Figures 7A as taken along a same sectional plane as in Figure 6D;
Figure 8 is an enlarged side view of the tapered end of the elongated rod member of a medical instrument in accordance with a variant of the embodiment shown in Figures 7A-B;
Figure 9A is a photographic illustration of sonographic imagery showing the terminal portion of the elongated member of a medical instrument that is provided with a marking in accordance with the invention, as shown e.g. in Figures 5A-D and 6A-E, which medical instrument is positioned in a first rotational orientation; and
Figures 9B and 9C are photographic illustrations of sonographic imagery showing the terminal portion of the elongated member of the medical instrument of Figure 9A positioned in two further rotational orientations.

### DETAILED DESCRIPTION OF EMBODIMENTS OF THE INVENTION

The present invention will be described in relation to various illustrative embodiments. It shall be understood that the scope of the invention encompasses all combinations and sub-combinations of the features of the medical instruments disclosed herein.

As described herein, when two or more parts or components are described as being connected, attached, secured or coupled to one another, they can be so connected, attached, secured or coupled directly to each other or through one or more intermediary parts.

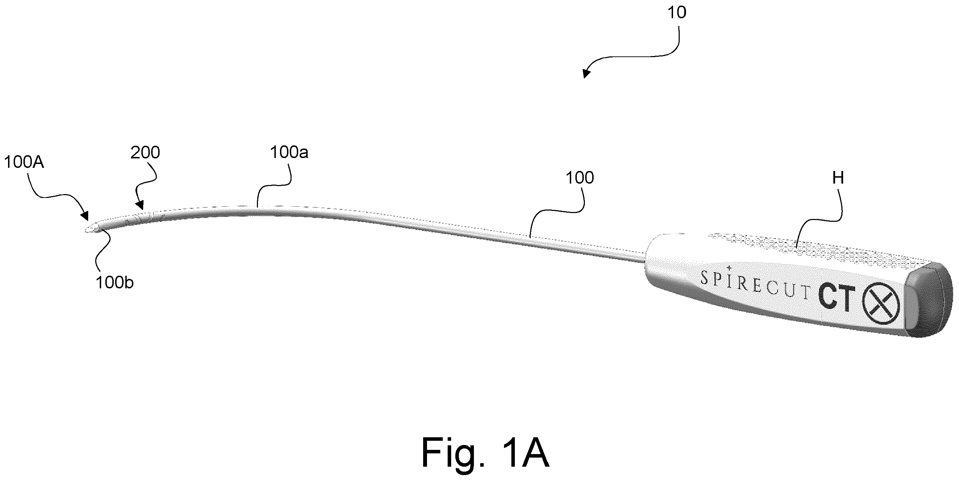

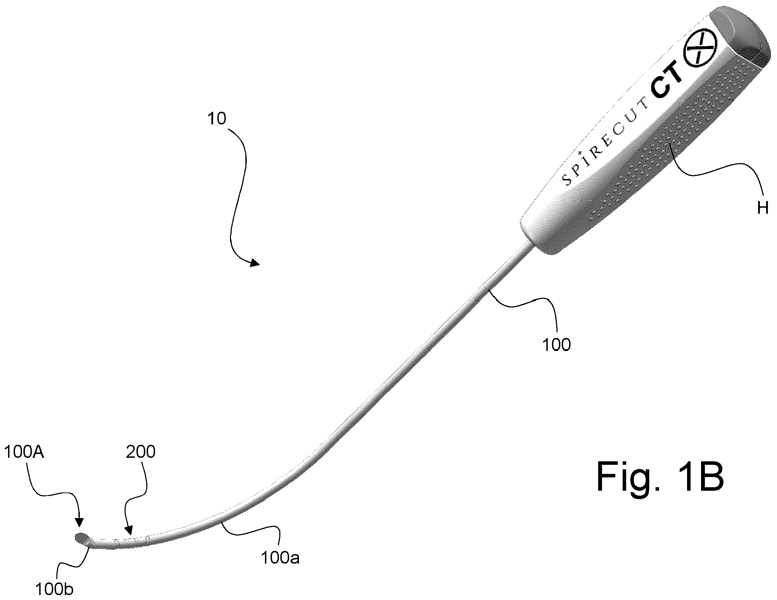

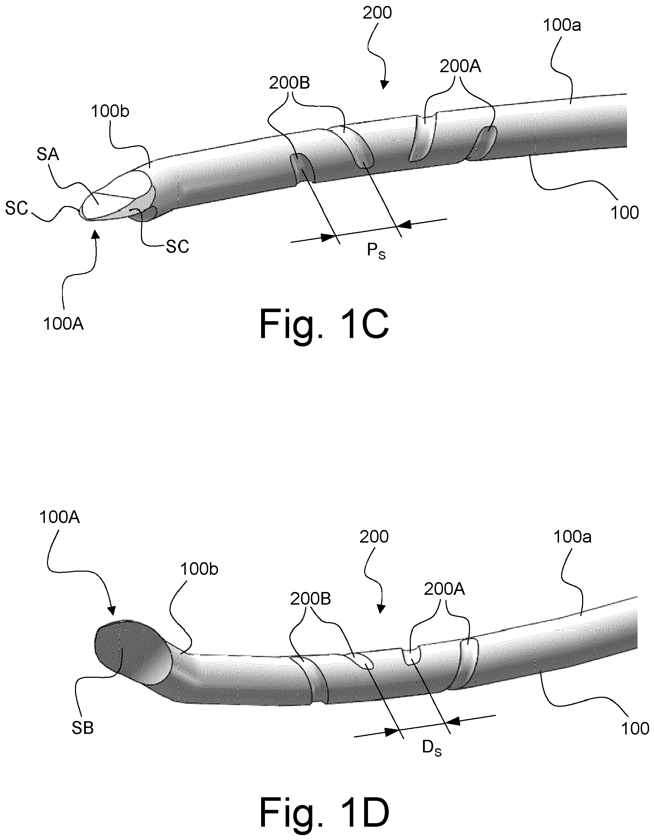

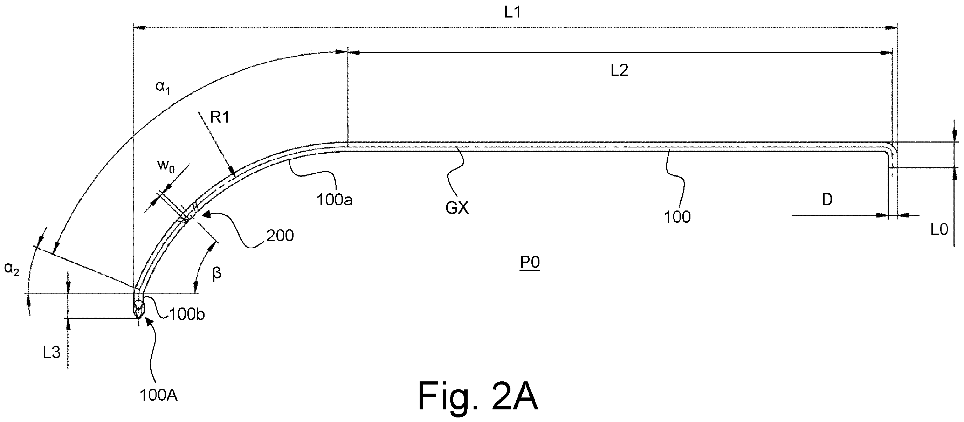

Referring to Figures 1A-D, there is shown a first embodiment of a medical instrument, designated by reference numeral 10, in accordance with the present invention, which first embodiment is particularly suited for percutaneous carpal tunnel release in the treatment of the carpal tunnel syndrome (CTS). Variants of this first embodiment are further shown in Figures 2A-E, 3A-B and 4.

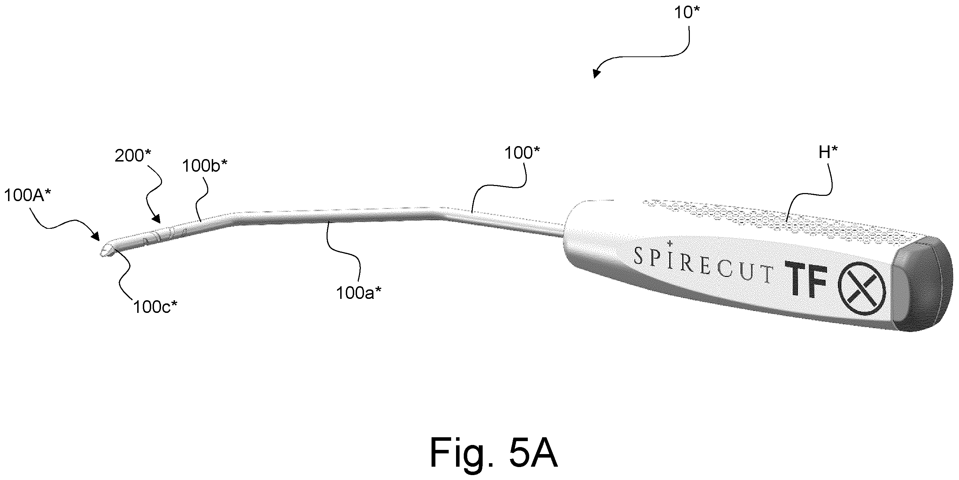

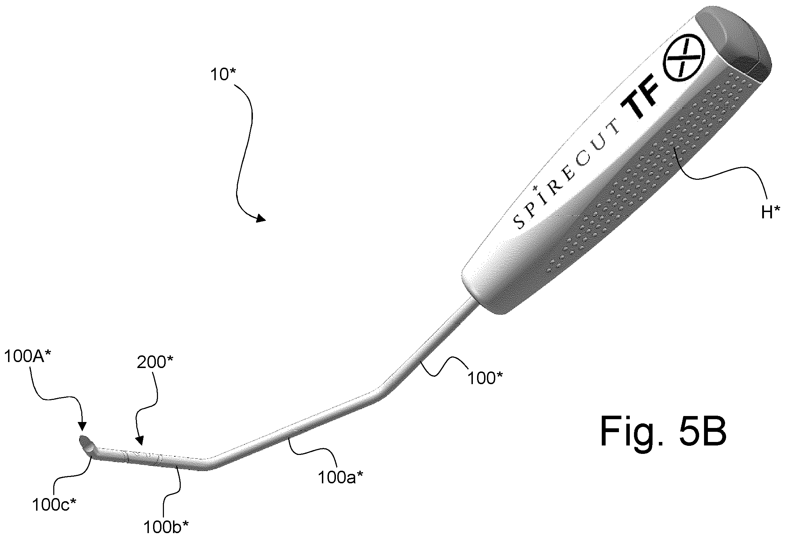

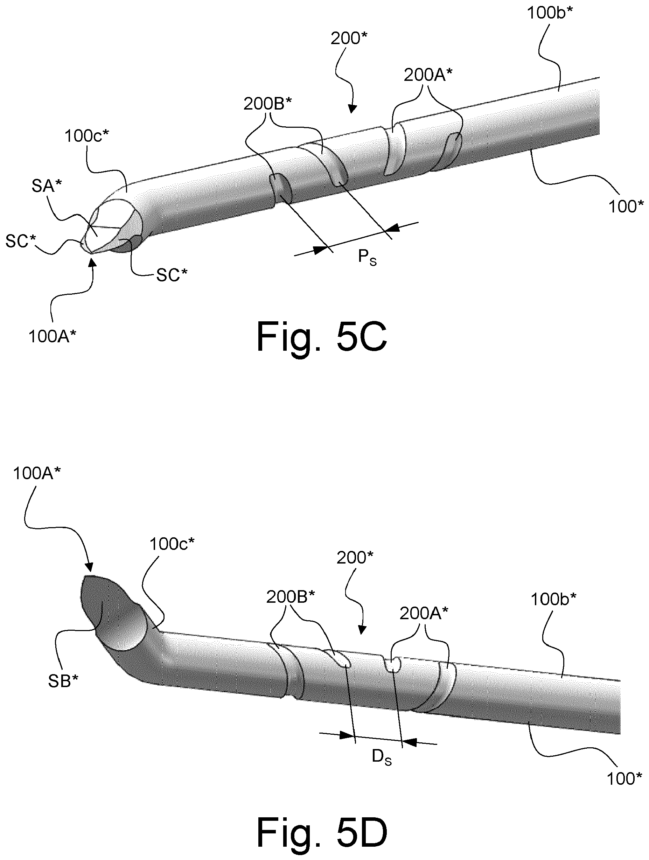

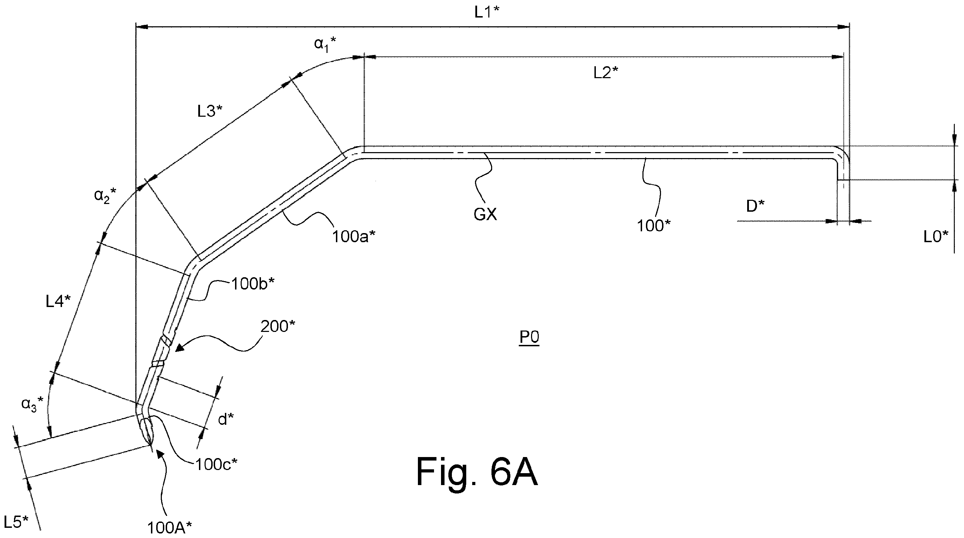

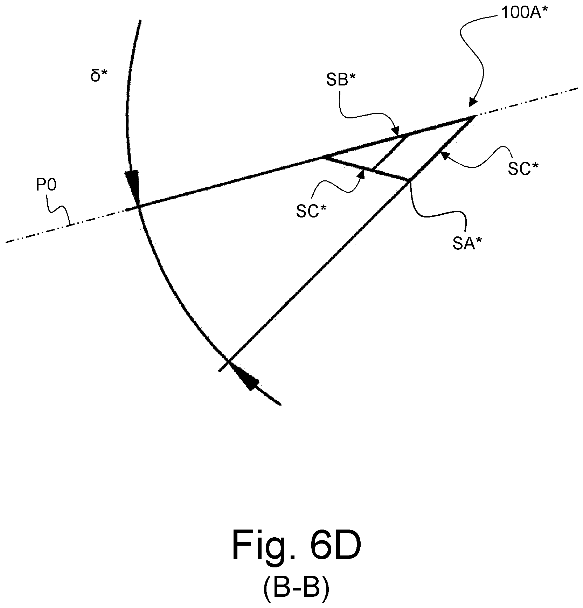

Referring to Figures 5A-D, there is shown another embodiment of a medical instrument, designated by reference numeral 10*, in accordance with the present invention, which other embodiment is particularly suited for percutaneous A1 pulley release in the treatment of the trigger finger syndrome (TFS). Variants of this second embodiment are further shown in Figures 6A-E, 7A-B and 8.

The medical instruments 10, 10* shown in the Figures are specifically designed to be used under sonography, i.e. in a combination with a sonography probe.

All embodiments share a number of common features, including a handle portion H, resp. H* (not shown in Figures 2A-E to 4 and 6A-E to 8) designed to allow handling, orientation and manipulation of the medical instrument by an operator, such as a surgeon or physician (including e.g. a radiologist or rheumatologist), and an elongated rod member 100, resp. 100*, secured to the handle portion H, resp. H* and extending therefrom up to a distal end. The elongated rod member 100, resp. 100* is curved and/or bent substantially within a defined plane, designated by reference P0.

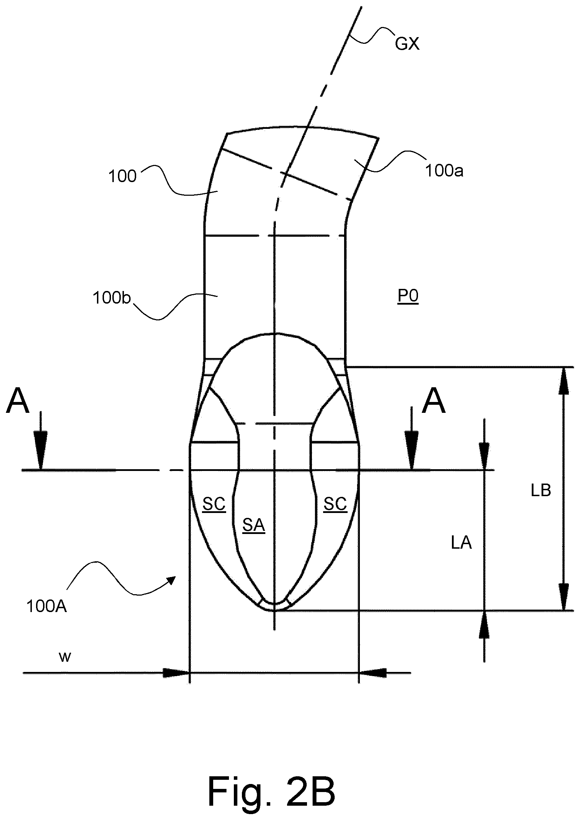

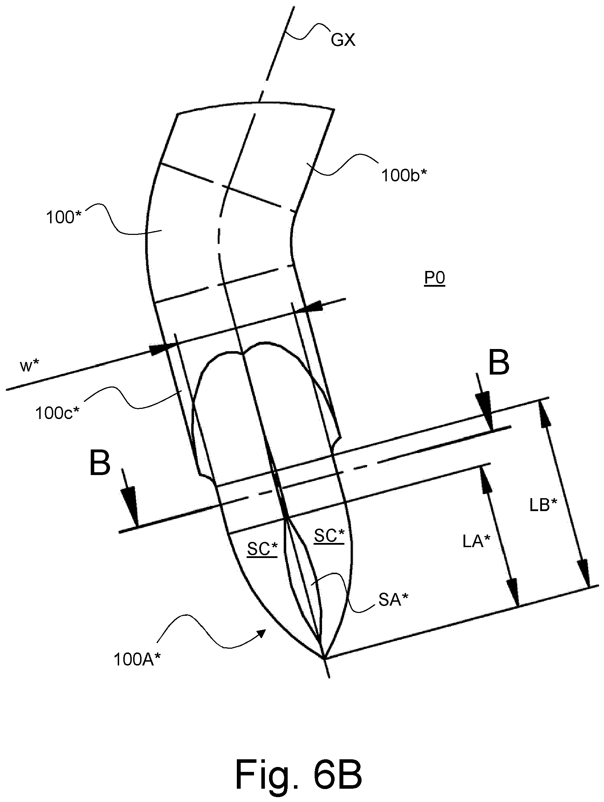

As shown in the illustrations, a first, rectilinear section of the elongated rod member 100, resp. 100*, extends substantially along a first direction within the defined plane P0 away from the handle portion H, resp. H*, namely parallel to the longitudinal axis of the handle portion H, resp. H*. The remainder of the elongated rod member 100, resp. 100*, downstream of the first rectilinear section, is curved and/or bent within the defined plane P0. Reference sign GX in Figures 2A, 2B, 6A and 6B designates a generatrix along which the elongated rod member 100, resp. 100*, extends. The free end of the first, rectilinear section of the elongated rod member 100, resp. 100* (see Figures 2A and 6A) is here bent at 90° to provide better retention within the handle portion H, resp. H*.

Furthermore, as this will be described in greater detail hereafter, the distal end of the elongated rod member 100, resp. 100*, comprises a tapered end 100A, 100A', 100A", 100A*, 100A** resp. 100A***, configured to act as a cutting device to sever tissue. The particular configuration of this tapered end 100A, 100A', 100A", 100A*, 100A** resp. 100A***, forms one aspect of the present invention.

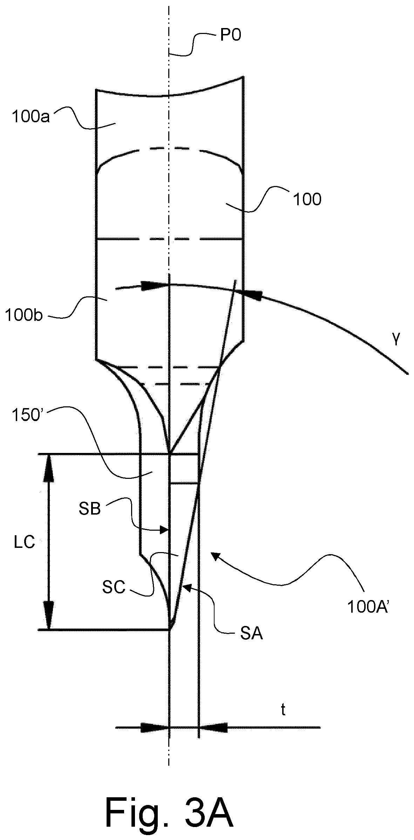

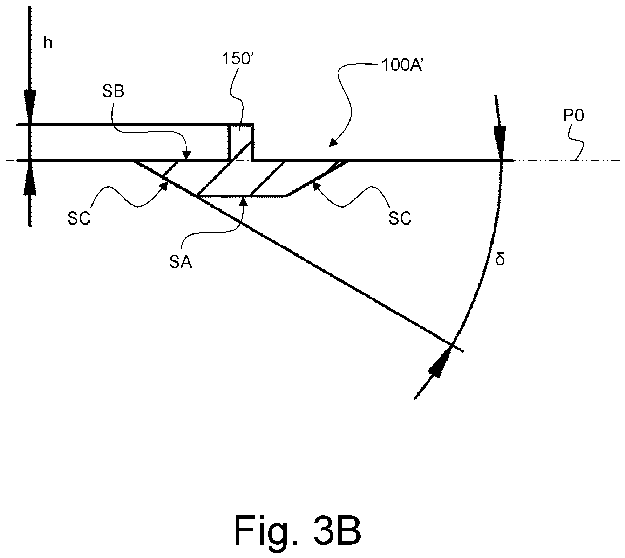

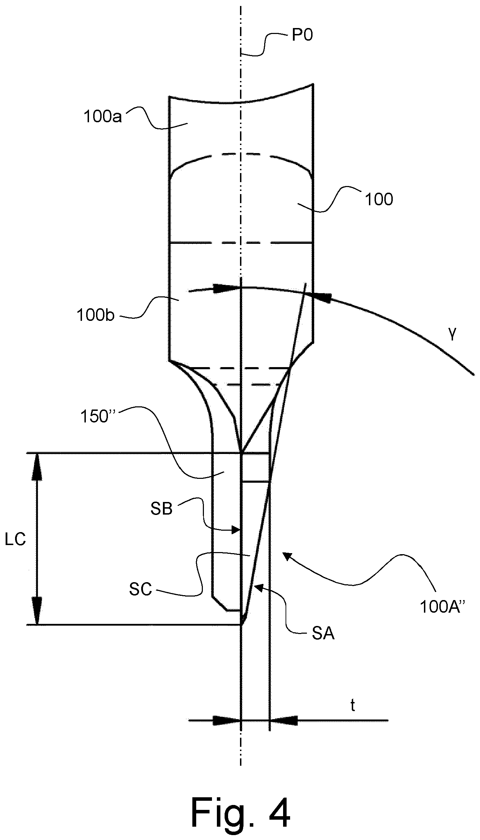

Referring more specifically to the embodiments of Figures 1A-D and 2A-E (which considerations apply to the variants shown in Figures 3A-B and 4), the elongated rod member 100 includes a curved section 100a extending over an angle α₁ (see Figure 2A which applies by analogy to the embodiments of Figures 1A-D, 3A-B and 4) that exceeds 60°. A radius of curvature R1 of the curved section 100a is of approximately 30 to 45 mm. By way of illustration, angle α₁ and radius of curvature R1 can be selected to equal approximately 68° and 37 mm, respectively, which values are not to be considered as limiting the scope of the invention. In this instance, it may be appreciated that the terminal section 100b of the elongated rod member 100 (including the aforementioned tapered end 100A, 100A', resp. 100A") is bent with respect to the end of the curved section 100a by an angle α₂ of approximately 20° (here 22°), so that the terminal section 100b extends substantially perpendicularly to the direction in which the first rectilinear section of the elongated rod member 100 extends (i.e. substantially perpendicularly to the longitudinal axis of the handle portion H).

By way of further illustration, an overall length L1 of the elongated rod member 100, as measured in the longitudinal direction (see Figure 2A) can be of approximately 125 mm. A length L2 of the first rectilinear section of the elongated rod member 100 can be of approximately 90 mm and a length L3 of the terminal section 100b of the elongated rod member 100 (including the tapered end 100A, 100A', resp. 100A") can be of approximately 4 mm. In the illustrated example, a length L0 of the bent portion at the free end of the first, rectilinear section of the elongated rod member 100 is of approximately 4 mm.

By way of preference, a lateral breadth of the elongated rod member 100 as measured at any point along the generatrix GX, up to an including the distal end of the elongated rod member 100, does not exceed 2 mm. More specifically, in the illustrated examples, the elongated rod member 100 preferably has a substantially circular cross-section, upstream of the distal end of the elongated rod member 100, whose diameter D does not exceed 1.5mm. Other cross-sectional shapes could however be contemplated, including triangular, square, rectangular, trapezoidal, hexagonal, and other polygonal cross-sections whose lateral breadth likewise preferably does not exceed 1.5 mm. A polygonal cross-section may be of advantage over a circular cross-section in that echogenicity is further improved.

More generally, a cross-sectional area of the elongated rod member 100 (including the tapered end 100A, 100A', resp. 100A") preferably does not exceed 2.5 mm². In other words, the elongated rod member 100 is of a particularly thin configuration, alike that of a puncture needle, which favours insertion and withdrawal of the medical instrument 10 without causing damage to surrounding tissues or structures.

The illustrated arc shape allows the medical instrument 10 to be introduced under or against the structure to be released into the sonography field without any interference between the handle portion of the instrument and the sonography probe all over the procedure. This shape also allows withdrawal of the medical instrument without risk of causing involuntary lesion to surrounding soft tissue.

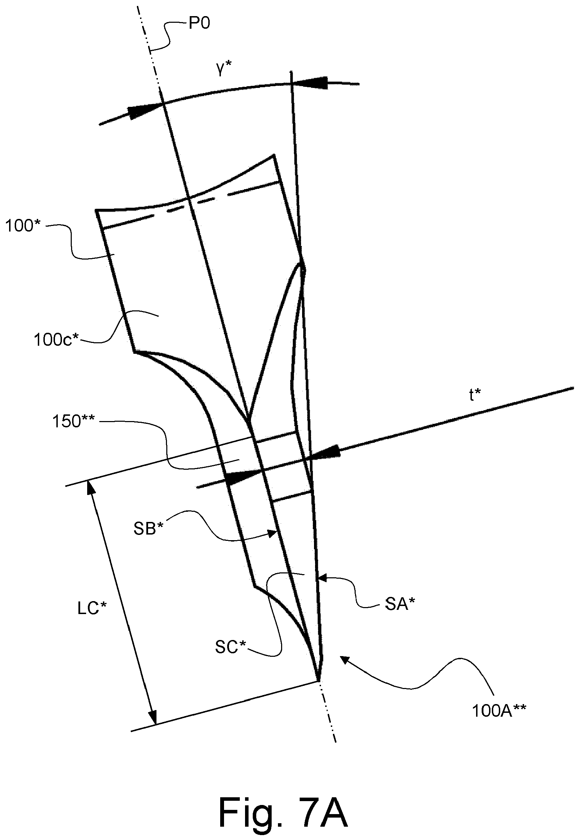

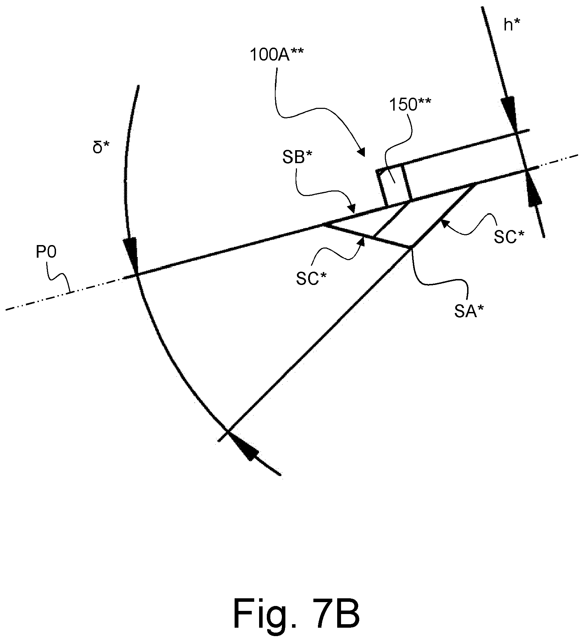

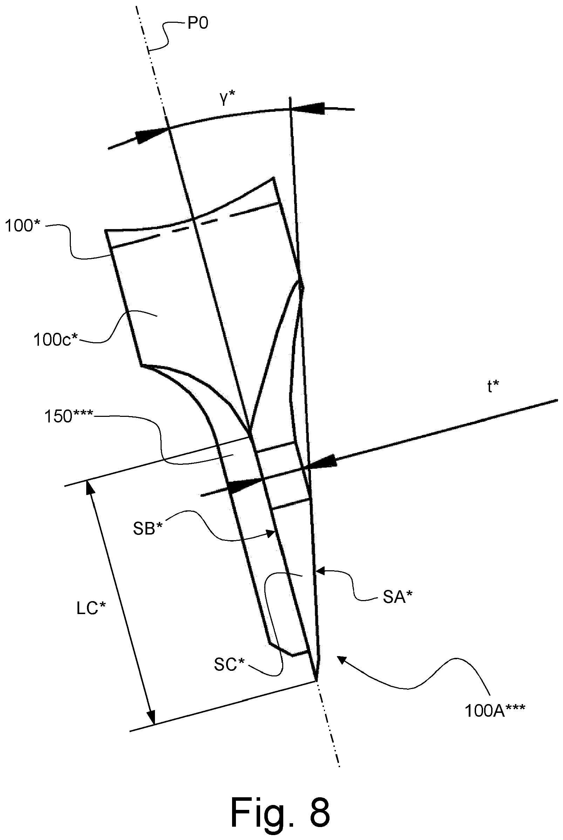

Referring to the embodiments of Figures 5A-D and 6A-E (which considerations apply to the variants shown in Figures 7A-B and 8), the elongated rod member 100* exhibits an angled configuration including three curved sections and three substantially rectilinear sections 100a*, 100b*, 100c* that are angled with respect to the first, rectilinear section of the elongated rod member 100*. In the illustrated example, each curved section has an angle α₁*, α₂*, α₃*, of approximately 35°, meaning that angled sections 100a*, 100b*, 100c* are angled at approximately 35°, 70° and 105°, respectively, with respect to the first, rectilinear section of the elongated rod member 100*. In other words, the terminal, angled section 100c* extends approximately perpendicularly, albeit a slightly rearward direction, with respect to the direction in which the first rectilinear section of the elongated rod member 100* extends (i.e. substantially perpendicularly to the longitudinal axis of the handle portion H*).

By way of further illustration, an overall length L1* of the elongated rod member 100*, as measured in the longitudinal direction (see Figure 6A) can be of approximately 90 mm. A length L2* of the first rectilinear section of the elongated rod member 100* can be of approximately 60 mm, while a length L3*, resp. L4* of angled sections 100a*, resp. 100b*, can be of approximately 20 mm. A length L5* of the terminal section 100c* of the elongated rod member 100* (including the tapered end 100A*, 100A**, resp. 100A***) can be of approximately 4 mm. In the illustrated example, a length L0* of the bent portion at the free end of the first, rectilinear section of the elongated rod member 100* is of approximately 4 mm.

By way of preference, a lateral breadth of the elongated rod member 100* as measured at any point along the generatrix GX, up to an including the distal end of the elongated rod member 100*, likewise does not exceed 2 mm. More specifically, in the illustrated examples, the elongated rod member 100* similarly has a substantially circular cross-section, upstream of the distal end of the elongated rod member 100*, whose diameter D* does not exceed 1.5 mm. Other cross-sectional shapes could likewise be contemplated, including polygonal cross-sections.

More generally, a cross-sectional area of the elongated rod member 100* (including the tapered end 100A*, 100A**, resp. 100A***) does not exceed 2.5 mm². In other words, the elongated rod member 100* is again of a particularly thin configuration, alike that of a puncture needle, which favours insertion and withdrawal of the medical instrument 10* without causing damage to surrounding tissues or structures.

The illustrated angled shape similarly allows the medical instrument 10* to be introduced under or against the structure to be released into the sonography field without any interference between the handle portion of the instrument and the sonography probe all over the procedure. This shape likewise also allows withdrawal of the medical instrument without risk of causing involuntary lesion to surrounding soft tissue.

One will therefore appreciate that parts of the curved or bent portions of the elongated rod member 100, resp. 100*, can especially be exploited during surgery as a fulcrum to facilitate severance of the desired tissue. In effect, parts of the curved or bent portions can suitably be used to bear on underlying tissues or onto an additional instrument inserted for that very purpose, thereby providing support for manipulation of the medical instrument during the severance operation.

It will also be appreciated that the medical instrument of the invention advantageously exhibits a minimal cross-section that in essence corresponds to the cross-section of the elongated rod member 100, resp. 100*, which greatly facilitates penetration through the tissues, with minimal interference both during insertion of the medical instrument in the area to be treated and during withdrawal thereof. Furthermore, the limited cross-sectional area of the elongated member (which by way of preference does not exceed 2.5 mm² as mentioned above) is such that surgery can be carried out in a truly percutaneous manner without necessitating any incision at the point of entry of the medical instrument, but merely a puncture, which therefore heals without any noticeable scar for the patient, much like after an intravenous infusion.

In accordance with an important aspect of the invention, as shown in Figures 1A-D, 2A, 2E, 5A-D, 6A and 6E, at least one portion of the elongated rod member 100, resp. 100*, proximate to the distal end, is provided with a marking 200, resp. 200*, that is distinguishable under sonography. More specifically, this marking 200, resp. 200*, is designed as a rotationally asymmetric marking configured to allow a location and a rotational orientation of the elongated rod member to be uniquely detectable in sonographic imagery. In effect, the marking 200, resp. 200*, is such that it produces a specific and particular echo in sonography imagery depending on the actual rotational orientation of the medical instrument in the sonographic field.

Referring to the embodiments of Figures 1A-D to 2A-E (which considerations likewise apply to the variants shown in Figures 3A-B and 4), the marking 200 is preferably positioned on the curved section 100a such that a central portion of the marking 200 is located at an angle β of approximately 45° with respect to the terminal section 100b of the elongated rod member 100, as shown in Figure 2A. Referring to the embodiments of Figures 5A-D to 6A-E (which considerations likewise apply to the variants shown in Figures 7A-B and 8), the marking 200* is preferably positioned on the second angled section 100b* so that a distance d* between the distal end of the second angled section 100b* and the marking 200* is of approximately 4 mm, as shown in Figure 6A. In this way, the marking 200, resp. 200*, will be visible in the sonographic imagery, along with the distal end of the elongated rod member 100, resp. 100*, thus allowing a location as well as a rotational orientation of the tip of the elongated rod member, and thus of the tapered end 100A, 100A', 100A", 100A* 100A**, resp. 100A***, to be identifiable and detectable.

Such detection can be performed visually by a trained operator, or even automatically or semi-automatically, using dedicated image processing software to properly identify relevant echoes/markers in the sonographic imagery. In that context, it could in particular be contemplated to use the relevant marking 200, resp. 200*, to automatically locate and detect the orientation of the medical instrument in the sonographic imagery, and provide visual assistance to the operator. This visual assistance could in particular include the superimposition in real time of a virtual representation of the medical instrument on the sonographic imagery, if need be.

By way of preference, as shown in greater detail in Figures 1C-D and 5C-D, the marking 200, resp. 200*, includes at least one rotationally asymmetric groove formed along a periphery of the elongated rod member 100, resp. 100*. In the illustrated example, two such grooves 200A, 200B, resp. 200A*, 200B*, are advantageously provided, which extend in opposite directions along the elongated rod member 100, resp. 100*. These grooves 200A, 200B, resp. 200A*, 200B*, are here in effect the mirrored image of one another.

More specifically, each asymmetric groove 200A, 200B, resp. 200A*, 200B*, consists of one helical turn, namely a single spire with an angle of development of approximately 360°. More than one helical turn could however be contemplated. By way of preference, a distance Ds separating a proximal end of the first groove 200A, resp. 200A*, and a proximal end of the second groove 200B, resp. 200B*, is substantially equal to a pitch Pₛ of each groove. In the illustrated examples, the distance Ds and pitch Pₛ are of approximately 2 mm.

Looking at the illustrated embodiments, especially Figures 1C-D and 5C-D, one will appreciate and understand that, depending on the actual rotational orientation of the tip of the elongated rod member 100, resp. 100*, with respect to the sonography probe, the grooves 200A, 200B, resp. 200A*, 200B*, will present two to four indentations to incoming ultrasonic waves produced by the sonography probe, thus producing different echoes, moreover with a unique and therefore clearly distinguishable spacing and/or distribution.

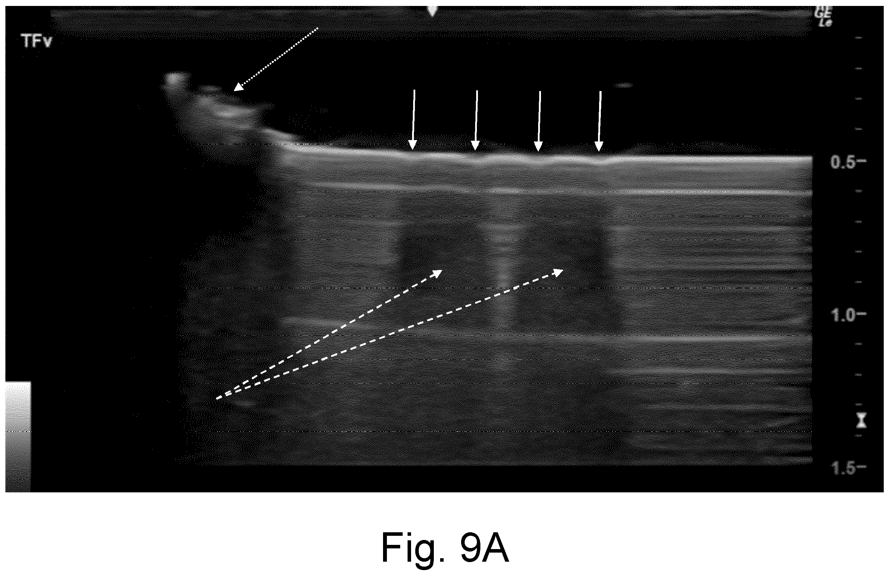

Figure 9A is a photographic illustration of sonographic imagery showing the terminal portion of the elongated member of a medical instrument that is provided with a marking in accordance with the invention, as shown e.g. in Figures 5A-D and 6A-E. Visible on the surface of the elongated rod member depicted in Figure 9A are four indentations (designated by continuous arrows) where the relevant grooves 200A*, 200B* are provided, as well as related "shadows" (designated by dashed arrows) in the sonographic imagery that betray the presence of the relevant grooves. These echoes and markers can be exploited to determine the actual rotational orientation of the medical instrument about its axis. Also visible in Figure 9A is the echo generated by the tapered end of the medical instrument (designated by a dotted arrow), which echo is further enhanced by the presence of a longitudinal ridge as discussed in greater detail hereafter.

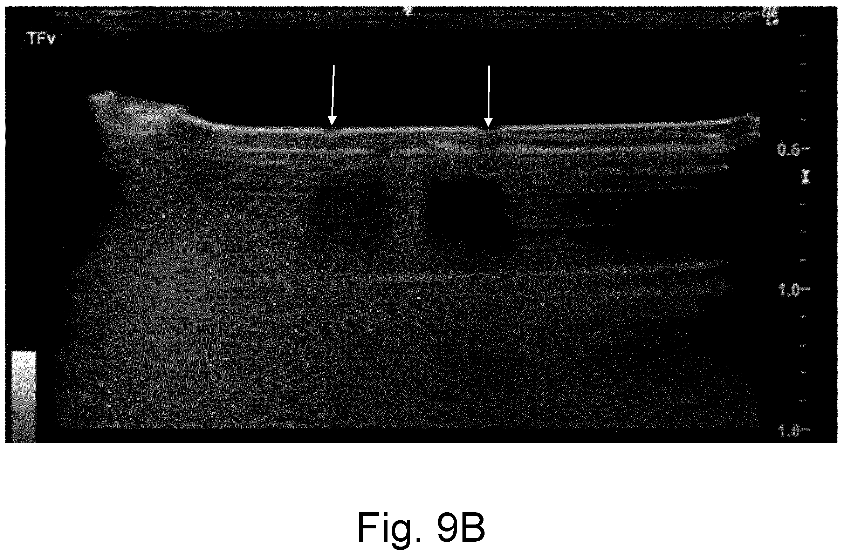

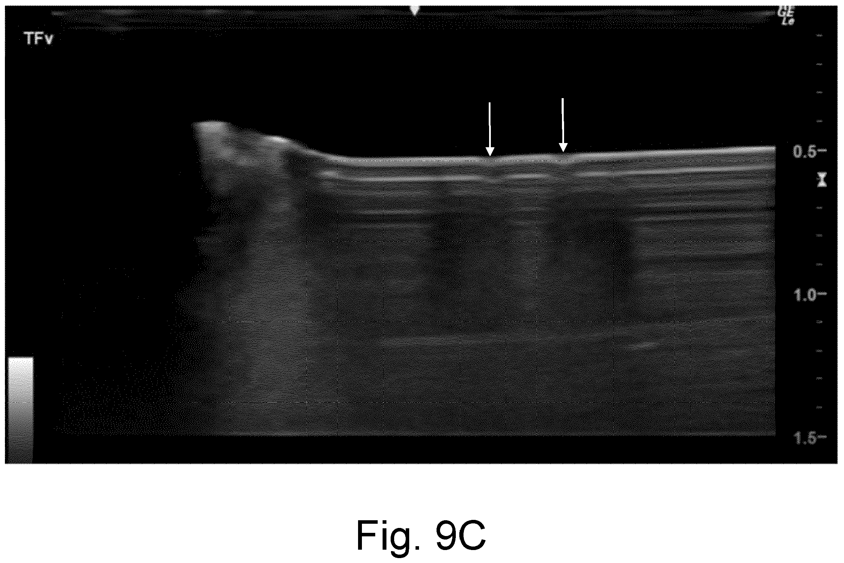

Figure 9B is a photographic illustration showing the terminal portion of the elongated member of the medical instrument of Figure 9A positioned in a different rotational orientation such that only two indentations (designated by continuous arrows) remain visible on the surface of the elongated rod member. The spacing between these indentations varies as a function of the rotational orientation of the medical instrument, as highlighted by Figure 9C which shows the medical instrument positioned in a further different rotational orientation.

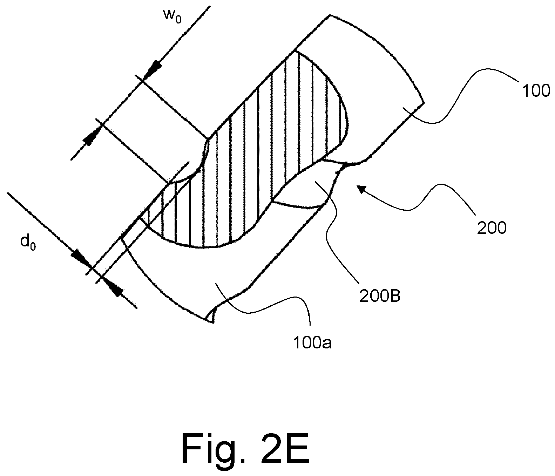

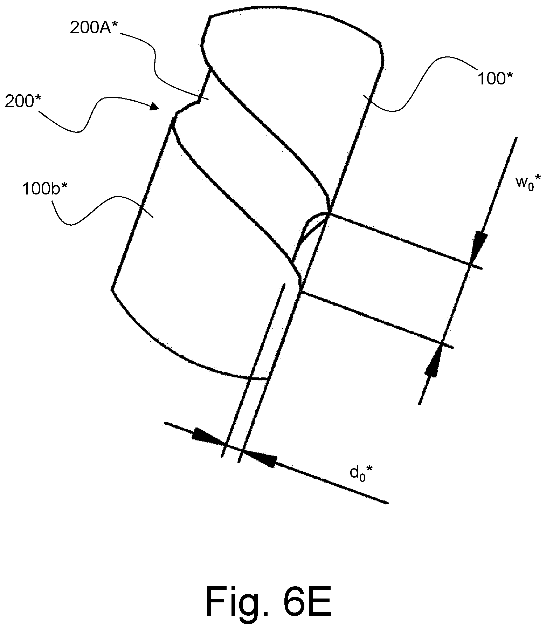

Each rotationally asymmetric groove 200A, 200B, resp. 200A*, 200B*, exhibits a width wo (see Figures 2A and 2E), resp. w₀* (see Figure 6E) that is preferably comprised between 0.5 mm and 1 mm. In the illustrated example, such width wo, w₀* is of approximately 0.6 mm to 0.7 mm. A depth of each groove is comparatively smaller and is advantageously comprised between 0.1 mm and 0.2 mm (see Figures 2E and 6E where such depth is designated by reference sign d₀, resp. d₀*).

In one embodiment, the depth of each rotationally asymmetric groove 200A, 200B, resp. 200A*, 200B*, may be constant. In another embodiment, the depth of each rotationally asymmetric groove 200A, 200B, resp. 200A*, 200B*, may advantageously vary along a length of the groove, which adds a further variable enhancing differentiated echogenicity depending on the actual rotational orientation of the marking in the sonographic field.

A further measure to enhance echogenicity may consist in structuring an inner surface of each rotationally asymmetric groove 200A, 200B, resp. 200A*, 200B*, as this favours dispersion of the ultrasonic waves hitting the inner surface of the grooves. Interestingly, structuring of the inner surface of each rotationally asymmetric groove 200A, 200B, resp. 200A*, 200B* may also favourably impact haptic feedback as the grooves 200A, 200B, resp. 200A*, 200B* may have a tendency to slightly cling on surrounding soft tissue, which also helps the operator to "feel" where the distal end of the medical instrument is located.

One may further play with the particular profile of each groove 200A, 200B, resp. 200A*, 200B* to cause distinctive dispersion of the ultrasonic waves hitting the inner surface of the grooves. Specifically, the groove profile could be essentially cylindrical or exhibit some asymmetry.

One will appreciate and understand that other marking configurations could be contemplated as long as the marking as a whole retains a rotationally asymmetric configuration. For instance, in the illustrated example, one of the two grooves 200A, 200B, resp. 200A*, 200B*, could perfectly be replaced by a simple annular groove, as the marking 200, resp. 200*, as a whole would still retain its rotational asymmetry. This being said, the solution illustrated in the Figures is favoured in that it is more easily identifiable in sonographic imagery.

A further aspect of the invention, applicable independently of the use of the aforementioned marking, will now be described with particular reference to Figures 1C-D, 2B-D, 3A-B, 4, 5C-D, 6B-D, 7A-B and 8, namely in relation to the particular shape of the tapered end 100A, 100A', 100A", 100A* 100A**, resp. 100A***, of the elongated rod member 100, resp. 100*. As illustrated, such tapered end 100A, 100A', 100A", 100A* 100A**, resp. 100A*** is at least partly delineated by first and second opposite surfaces SA, SB, resp. SA*, SB*, namely a first, bevelled surface SA, resp. SA*, that is inclined with respect to the defined plane P0 and a second, recessed surface SB, resp. SB*, that is substantially parallel to the defined plane P0. By way of preference, as shown, the tapered end 100A, 100A', 100A", 100A* 100A**, resp. 100A***, is further delineated by a pair of side surfaces SC, resp. SC*, forming side bevels (see especially Figures 1C, 2B-D, 3A-B, 4, 5C, 6B-D, 7A-B and 8) alongside at least a portion of the bevelled surface SA, resp. SA*, and of the recessed surface SB, resp. SB*. In this way, particularly sharp cutting edges can be achieved at the leading and side edges of the resulting tapered end 100A, 100A', 100A", 100A* 100A**, resp. 100A***

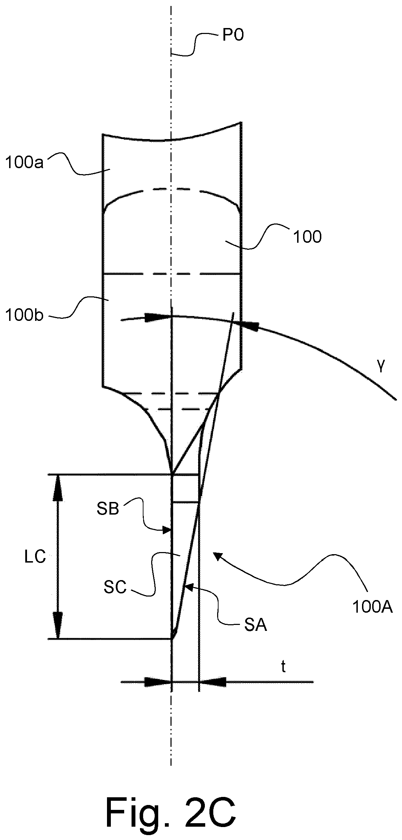

Referring more specifically to Figures 2B-D (which considerations apply by analogy to the variants shown in Figures 3A-B and 4), a length LA of the bevelled surface SA and a length LB of the tapered tip of the elongated rod member 100, as measured within defined plane P0 (see Figure 2B) are of approximately 1.5 mm and 2.6 mm, respectively, in the illustrated example. A width w of the tapered end 100A, 100A', resp. 100A", as likewise measured within the defined plane P0, is here, by way of illustration, of approximately 1.8 mm, i.e. slightly larger than the relevant diameter D of the elongated rod member 100. Ideally, such width w should not exceed 2 mm. A length LC of the recessed surface SB (and of the tapered end 100A, 100A', resp. 100A" as such) is of approximately 1.8 mm in the illustrated example, while a thickness t of the tapered end 100A, 100A', resp. 100A", as measured perpendicularly to the defined plane P0, is of approximately 0.3 mm (see Figure 2C, 3A and 4).

Figures 3A-B and 4 show variants of the tapered end, designated by reference signs 100A' and 100A" respectively, of the elongated rod member 100 that are aimed at further improving echogenicity of the tapered end 100A', resp. 100A". The main difference compared to the embodiments shown in Figures 1A-D and 2A-E resides in the fact that the tapered end 100A', resp. 100A" further includes a longitudinal ridge 150', resp. 150" projecting away from the recessed surface SB. A height h of this longitudinal ridge 150', resp. 150" as measured perpendicularly to defined plane P0 preferably does not exceed 0.5 mm. In the illustrated example, the height h is of approximately 0.3 mm. The width of the longitudinal ridge 150', resp. 150" as measured within defined plane P0 is only a fraction (e.g. 10% to 20%) of the width w of the tapered end 100A', resp. 100A".

Tests carried by the Applicant have demonstrated that the provision of the longitudinal ridge 150', resp. 150" further improves echogenicity of the tapered end 100A', resp. 100A" under sonography.

One will appreciate that the presence of the longitudinal ridge 150', resp. 150" on the recessed surface SC causes a local increase of the thickness of the tapered end 100A', resp. 100A" compared to that of the tapered end 100A shown in Figures 1A-D and 2A-E. This being said, the dimensions of the longitudinal ridge 150', resp. 150" are selected such as to minimize any interference during insertion of the medical instrument in the area to be treated. Furthermore, the leading edge of the longitudinal ridge 150', resp. 150" is preferably tapered to further minimize any interference during insertion of the medical instrument. One may also further note that the leading edge of the longitudinal ridge 150', resp. 150" stops short of the leading edge of the tapered end 100A', resp. 100A" in order not to impact the cutting properties and sharpness thereof. Figures 3A and 4 for instance show the provision of a suitable curved section (Figure 3A) or chamfered section (Figure 4) at the leading edge of the longitudinal ridge 150', resp. 150".

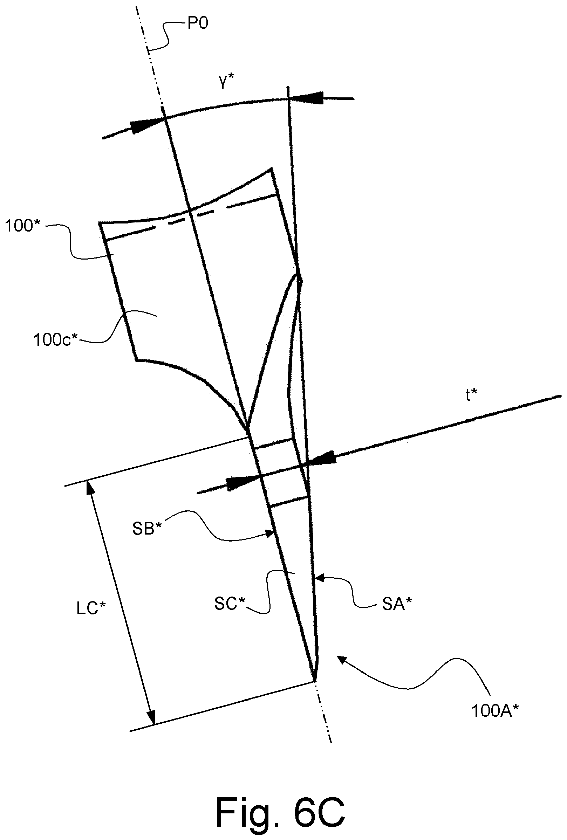

Referring more specifically to Figures 6B-D (which considerations apply to the variants shown in Figures 7A-B and 8), a length LA* of the bevelled surface SA* and a length LB* of the tapered tip of the elongated rod member 100*, as measured within defined plane P0 (see Figure 6B) are of approximately 1.5 mm and 2 mm, respectively, in the illustrated example. A width w* of the tapered end 100A* 100A**, resp. 100A***, as likewise measured within the defined plane P0, is here, by way of illustration, of approximately 1.25 mm, i.e. slightly smaller than the relevant diameter D* of the elongated rod member 100*. Ideally, such width w* should likewise not exceed 2 mm. A length LC* of the recessed surface SB* (and of the tapered end 100A*, 100A**, resp. 100A*** as such) is of approximately 2 mm in the illustrated example, while a thickness t* of the tapered end 100A*, 100A**, 100A***, as measured perpendicularly to the defined plane P0, is of approximately 0.35 mm (see Figure 6C, 7A and 8).

Figures 7A-B and 8 show variants of the tapered end, designated by reference signs 100A** and 100A*** respectively, of the elongated rod member 100* that are aimed at further improving echogenicity of the tapered end 100A**, resp. 100A***. The main difference compared to the embodiments shown in Figures 5A-D and 6A-E resides in the fact that the tapered end 100A**, resp. 100A*** further includes a longitudinal ridge 150**, resp. 150*** projecting away from the recessed surface SB*, A height h* of this longitudinal ridge 150**, resp. 150*** as measured perpendicularly to defined plane P0 preferably does not exceed 0.5 mm. In the illustrated example, the height h* is of approximately 0.3 mm. The width of the longitudinal ridge 150**, resp. 150*** as measured within defined plane P0 is only a fraction (e.g. 10% to 20%) of the width w* of the tapered end 100A**, resp. 100A***.

Tests carried by the Applicant have again demonstrated that the provision of the longitudinal ridge 150**, resp. 150*** likewise further improves echogenicity of the tapered end 100A**, resp. 100A*** under sonography.

One will again appreciate that the presence of the longitudinal ridge 150**, resp. 150*** on the recessed surface SC* causes a local increase of the thickness of the tapered end 100A**, resp. 100A*** compared to that of the tapered end 100A* shown in Figures 5A-D and 6A-E. This being said, the dimensions of the longitudinal ridge 150**, resp. 150*** are likewise selected such as to minimize any interference during insertion of the medical instrument in the area to be treated. Furthermore, the leading edge of the longitudinal ridge 150**, resp. 150*** is preferably tapered to further minimize any interference during insertion of the medical instrument. One may likewise further note that the leading edge of the longitudinal ridge 150**, resp. 150*** stops short of the leading edge of the tapered end 100A**, resp. 100A*** in order not to impact the cutting properties and sharpness thereof. Figures 7A and 8 for instance show the provision of a suitable curved section (Figure 7A) or chamfered section (Figure 8) at the leading edge of the longitudinal ridge 150**, resp. 150***.

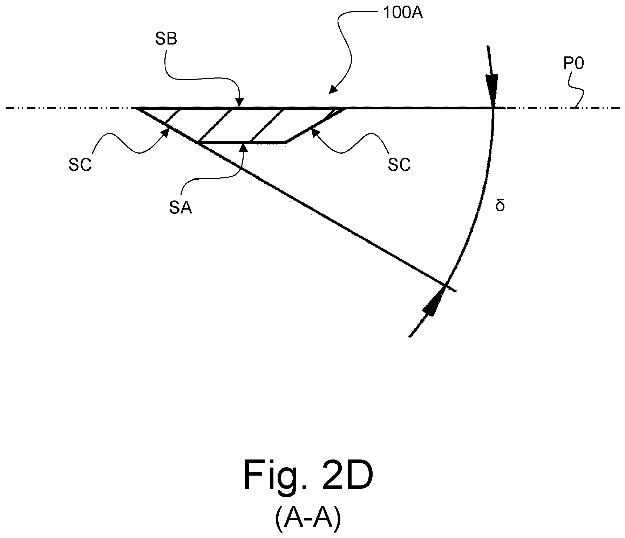

An angle of inclination γ, resp. γ*, of a plane comprising the bevelled surface SA, resp. SA*, with respect to the defined plane P0 (see Figures 2C, 3A, 4, 6C, 7A and 8) is preferably comprised between 10° and 15°, while an angle of inclination δ, resp. δ*, of side surfaces SC, resp. SC*, with respect to the defined plane P0 (i.e. with respect to the recessed surface SB, resp. SB* - see Figures 2D, 3B, 4D and 7B) is preferably comprised between 25° and 35°.

As this will be appreciated from reading the foregoing description, the medical instrument of the invention is of simple construction and is therefore reasonably economical to produce.

Advantageously, as illustrated in Figures 1A-B and 5A-B, a visible marking (such as a laser marking) may be provided on the handle portion H, resp. H*, to identify an orientation of the medical instrument when performing surgery, i.e. when the distal end of the medical instrument has been inserted into the area to be treated. This visible marking may in particular be provided on an inner face of the handle portion H, resp. H*, which is oriented in a same direction as the tapered end 100A, 100A', 100A", 100A*, 100A**, resp. 100A***

In the illustrations of Figures 1A-D to 8, one may appreciate that the elongated rod member 100, resp. 100*, is shown as being solid and non-hollow. One may however possibly contemplate to use a hollow rod member instead, which would provide for the ability to detect a possible bleeding through the relevant rod cavity, in case of damage to a blood vessel, and/or to inject a local anaesthetic (or any other drug like steroids or corticosteroids) using a dedicated syringe inserted in the relevant rod cavity. In this case, it is advantageous to further adapt the handle portion H, resp. H*, of the medical instrument to likewise exhibit a corresponding cavity communicating with the rod cavity to e.g. allow injection of the local anaesthetic or even a window allowing observation of a potential reflux of blood.

As already mentioned, the medical instrument of the invention can in particular be used for the purpose of performing percutaneous release procedures on upper or lower limbs, especially under the assistance of a sonography. Other uses could however be contemplated. Furthermore, the provision of the relevant marking discussed above could be contemplated on medical instruments that are not necessarily designed for the purpose of performing percutaneous release procedures, and could be applied to any medical instrument designed for the purpose of carrying out any other surgical or medical procedure, such as e.g. biopsy probes or devices designed to remove foreign bodies.

As regards the relevant surgical procedures for release of the transverse carpal ligament (TCL) using e.g. the medical instrument 10 discussed above or for release of the A1 pulley using e.g. the medical instrument 10* discussed above, such surgical procedures can be carried out in essentially the same manner as already disclosed in International (PCT) Publication No. WO 2020/003263 A1 in the name of the present Applicant.

Various modifications and/or improvements may be made to the above-described embodiments without departing from the scope of the invention as defined by the appended claims. In particular, while embodiments of the invention have been described for the purpose of carrying out percutaneous carpal tunnel release and percutaneous A1 pulley release, the invention is generally applicable to any percutaneous release procedure, be it on upper or lower limbs, or other parts of the body, such as the neck or spine.

With regard to the configuration of the relevant marking that is distinguishable under sonography, one will appreciate that such marking make take any other suitable shape or structure and does not necessarily need to be comprised of one or more grooves formed in the periphery of the elongated rod member as illustrated in the drawings. The marking may for instance comprise a series of indentations distributed non-uniformly about a portion of the circumference of the elongated rod member or any other suitable structuration or pattern providing rotational asymmetry to the marking. The machining of grooves however remains a cost-efficient solution to create the desired marking.

Furthermore, while the embodiments of Figures 1A-D to 8 show medical instruments where the tapered end exhibits strictly planar surfaces, the claims shall be construed as encompassing all variants where each surface is substantially planar or exhibits a slight curvature, be it slightly concave or convex. By way of convention, the plane of the relevant surfaces can generally be defined as a plane that best approximates each surface being considered.

In addition, while the cross-section of the longitudinal ridge 150', 150", 150**, resp. 150*** is shown as being substantially rectangular (which gives very good results), such cross-section could alternatively be V-shaped, trapezoidal, rounded, or exhibit any other suitable shape to enhance echogenicity of the tapered end of the medical instrument.

### LIST OF REFERENCE NUMERALS AND SIGNS USED THEREIN

- 10: medical instrument according to the invention (first embodiment)
- H: handle portion of medical instrument 10
- 100: elongated rod member of medical instrument 10 extending within defined plane P0
- 100a: (single) curved section of elongated rod member 100
- 100b: terminal section of elongated rod member 100 including tapered end 100A, 100A', resp. 100A"
- 100A: tapered end of elongated rod member 100 designed to act as a cutting device to severe tissue (Figures 1A-D to 2A-E)
- 100A': tapered end of elongated rod member 100 designed to act as a cutting device to severe tissue (variant of Figures 3A-B)
- 100A": tapered end of elongated rod member 100 designed to act as a cutting device to severe tissue (variant of Figure 4)
- 150': longitudinal ridge projecting away from recessed surface SB (variant of Figures 3A-B)
- 150": longitudinal ridge projecting away from recessed surface SB (variant of Figure 4)
- 200: rotationally asymmetric marking distinguishable under sonography
- 200A: (first) rotationally asymmetric groove formed along a periphery of the elongated rod member 100, proximate to the distal end thereof
- 200B: (second) rotationally asymmetric groove formed along a periphery of the elongated rod member 100, proximate to the distal end thereof
- SA: bevelled surface (first surface) delineating a first side of tapered end 100A, 100A', resp. 100A" / surface inclined with respect to defined plane P0
- SB: recessed surface (second surface) delineating a second side of tapered end 100A, 100A', resp. 100A", opposite the first side / surface substantially parallel to defined plane P0
- SC: pair of side surfaces forming side bevels alongside at least a portion of the bevelled surface SA and of the recessed surface SB
- D: diameter (or lateral breadth) of elongated rod member 100
- L0: length of bent portion at proximal end of elongated rod member 100
- L1: overall length of elongated rod member 100
- L2: length of (first) rectilinear section of elongated rod member 100
- L3: length of terminal section 100b (including tapered end 100A, 100A', resp. 100A")
- LA: approximate length, as measured within defined plane P0, of bevelled surface SA
- LB: approximate length, as measured within defined plane P0, of tapered tip of elongated rod member 100
- LC: approximate length, as measured within defined plane P0, of recessed surface SB / tapered end 100A, 100A', resp. 100A"
- h: approximate height, as measured perpendicularly to defined plane P0, of longitudinal ridge 150', resp. 150"
- R1: radius of curvature of curved section 100a
- α₁: angle of curved section 100a
- α₂: angle of bent portion at the junction between curved section 100a and terminal section 100b
- β: angle between terminal section 100b and central portion of marking 200
- γ: angle of inclination of bevelled surface SA with respect to defined plane P0
- δ: angle of inclination of side surfaces SC with respect to defined plane P0 / recessed surface SB
- w: width of tapered end 100A, 100A', resp. 100A", as measured within defined plane P0
- t: thickness of tapered end 100A, as measured perpendicularly to defined plane P0 / thickness of tapered end 100A', resp. 100A", as measured perpendicularly to defined plane P0 (excluding longitudinal ridge 150', resp. 150")
- wo: width of rotationally asymmetric groove 200A, resp. 200B
- 10*: medical instrument according to the invention (second embodiment)
- H*: handle portion of medical instrument 10*
- 100*: elongated rod member of medical instrument 10* extending within defined plane P0
- 100a*: (first) angled section of elongated rod member 100*
- 100b*: (second) angled section of elongated rod member 100*
- 100c*: (third) angled section of elongated rod member 100* (terminal section of elongated rod member 100* including tapered end 100A*, 100A**, resp. 100A***)
- 100A*: tapered end of elongated rod member 100* designed to act as a cutting device to severe tissue
- 100A**: tapered end of elongated rod member 100 designed to act as a cutting device to severe tissue (variant of Figures 7A-B)
- 100A***: tapered end of elongated rod member 100 designed to act as a cutting device to severe tissue (variant of Figure 8)
- 150**: longitudinal ridge projecting away from recessed surface SB* (variant of Figures 7A-B)
- 150***: longitudinal ridge projecting away from recessed surface SB* (variant of Figure 8)
- 200*: rotationally asymmetric marking distinguishable under sonography
- 200A*: (first) rotationally asymmetric groove formed along a periphery of the elongated rod member 100*, proximate to the distal end thereof
- 200B*: (second) rotationally asymmetric groove formed along a periphery of the elongated rod member 100*, proximate to the distal end thereof
- SA*: bevelled surface (first surface) delineating a first side of tapered end 100A*, 100A**, resp. 100A*** / surface inclined with respect to defined plane P0
- SB*: recessed surface (second surface) delineating a second side of tapered end 100A*, 100A**, resp. 100A***, opposite the first side / surface substantially parallel to defined plane P0
- SC*: pair of side surfaces forming side bevels alongside at least a portion of the bevelled surface SA* and of the recessed surface SB*
- D*: diameter (or lateral breadth) of elongated rod member 100*
- L0*: length of bent portion at proximal end of elongated rod member 100*
- L1*: overall length of elongated rod member 100*
- L2*: length of (first) rectilinear section of elongated rod member 100*
- L3*: length of (first) angled section 100a*
- L4*: length of (second) angled section 100b*
- L5*: length of (third) angled section 100c*
- LA*: approximate length, as measured within defined plane P0, of bevelled surface SA*
- LB*: approximate length, as measured within defined plane P0, of tapered tip of elongated rod member 100*
- LC*: approximate length, as measured within defined plane P0, of recessed surface SB*/ tapered end 100A* 100A**, resp. 100A***
- h*: approximate height, as measured perpendicularly to defined plane P0, of longitudinal ridge 150**, resp. 150***
- d*: distance between distal end of (second) angled section 100b* and marking 200*
- α₁*: angle of curved section between (first) rectilinear section of elongated rod member 100* and (first) angled section 100a*
- α₂*: angle of curved section between (first) angled section 100a* and (second) angled section 100b*
- α₃*: angle of curved section between (second) angled section 100b* and (third) angled section 100c*
- γ*: angle of inclination of bevelled surface SA* with respect to defined plane P0
- δ*: angle of inclination of side surfaces SC* with respect to defined plane P0 / recessed surface SB*
- w*: width of tapered end 100A*, 100A**, resp. 100A***, as measured within defined plane P0
- t*: thickness of tapered end 100A*, as measured perpendicularly to defined plane P0 / thickness of tapered end 100A**, resp. 100A***, as measured perpendicularly to defined plane P0 (excluding longitudinal ridge 150**, resp. 150***)
- w₀*: width of rotationally asymmetric groove 200A*, resp. 200B*
- d₀*: depth of rotationally asymmetric groove 200A*, resp. 200B*
- Ds: distance separating a proximal end of the first rotationally asymmetric groove 200A, resp. 200A*, and a proximal end of the second rotationally asymmetric groove 200B, resp. 200B*
- Pₛ: pitch of helical turn forming rotationally asymmetric groove 200A, 200B, 200A*, resp. 200B*
- GX: generatrix along which the elongated rod member 100, resp. 100*, extends
- P0: defined plane in which the elongated rod member 100, resp. 100*, extends

## Claims

1. A medical instrument (10; 10*), in particular for percutaneous surgical/medical procedures, comprising a handle portion (H; H*) designed to allow handling, orientation and manipulation of the medical instrument (10; 10*) by an operator and an elongated rod member (100; 100*) secured to the handle portion (H; H*),
wherein the elongated rod member (100; 100*) extends from the handle portion (H; H*) up to a distal end and is preferably curved and/or bent substantially within a defined plane (P0),
wherein at least one portion of the elongated rod member (100; 100*), proximate to the distal end, is provided with a marking (200; 200*) distinguishable under sonography,
**characterized in that** the marking (200; 200*) is designed as a rotationally asymmetric marking configured to allow a location and a rotational orientation of the elongated rod member (100; 100*) to be uniquely detectable in sonographic imagery.

2. The medical instrument (10; 10*) according to claim 1, wherein the marking (200; 200*) includes at least one rotationally asymmetric groove (200A, 200B; 200A*, 200B*) formed along a periphery of the elongated rod member (100; 100*).

3. The medical instrument (10; 10*) according to claim 2, wherein the marking (200; 200*) includes first and second rotationally asymmetric grooves (200A, 200B; 200A* 200B*) extending in opposite directions along the elongated rod member (100; 100*).

4. The medical instrument (10; 10*) according to claim 2 or 3, wherein each rotationally asymmetric groove (200A, 200B; 200A* 200B*) consists of one or more helical turns.

5. The medical instrument (10; 10*) according to claim 3, wherein each rotationally asymmetric groove (200A, 200B; 200A*, 200B*) consists of one or more helical turns,
wherein a distance (Ds) separating a proximal end of the first rotationally asymmetric groove (200A; 200A*) and a proximal end of the second rotationally asymmetric groove (200B; 200B*) is substantially equal to a pitch (Pₛ) of each rotationally asymmetric groove (200A, 200B; 200A*, 200B*),
and wherein said distance (Ds) and said pitch (Pₛ) are preferably of approximately 2 mm.

6. The medical instrument (10; 10*) according to any one of claims 2 to 5, wherein each rotationally asymmetric groove (200A, 200B; 200A*, 200B*) exhibits a width (wo; w₀*) comprised between 0.5 mm and 1 mm and/or a depth (d₀*) comprised between 0.1 mm and 0.2 mm.

7. The medical instrument (10; 10*) according to any one of claims 2 to 6, wherein a depth of each rotationally asymmetric groove (200A, 200B; 200A* 200B*) is constant or varies along a length of the rotationally asymmetric groove (200A, 200B; 200A*, 200B*).

8. The medical instrument (10; 10*) according to any one of claims 2 to 7, wherein an inner surface of each rotationally asymmetric groove (200A, 200B; 200A*, 200B*) is structured.

9. The medical instrument (10; 10*) according to any one of the preceding claims, wherein the distal end of the elongated rod member (100; 100*) comprises a tapered end (100A; 100A'; 100A"; 100A*; 100A**; 100A***) configured to act as a cutting device to sever tissue,
and wherein the tapered end (100A; 100A'; 100A"; 100A*; 100A**; 100A***) is at least partly delineated by first and second opposite surfaces (SA, SB; SA*, SB*), namely a first, bevelled surface (SA; SA*) that is inclined with respect to the defined plane (P0) and a second, recessed surface (SB; SB*) that is substantially parallel to the defined plane (P0).

10. A medical instrument (10; 10*) for percutaneous release procedures, comprising a handle portion (H; H*) designed to allow handling, orientation and manipulation of the medical instrument (10; 10*) by an operator and an elongated rod member (100; 100*) secured to the handle portion (H; H*),
wherein the elongated rod member (100; 100*) extends from the handle portion (H; H*) up to a distal end and is curved and/or bent substantially within a defined plane (P0),
wherein the distal end of the elongated rod member (100; 100*) comprises a tapered end (100A; 100A'; 100A"; 100A*; 100A**; 100A***) configured to act as a cutting device to sever tissue,
**characterized in that** the tapered end (100A; 100A'; 100A'; 100A*; 100A**; 100A***) of the elongated rod member (100; 100*) is at least partly delineated by first and second opposite surfaces (SA, SB; SA*, SB*), namely a first, bevelled surface (SA; SA*) that is inclined with respect to the defined plane (P0) and a second, recessed surface (SB; SB*) that is substantially parallel to the defined plane (P0).

11. The medical instrument (10; 10*) according to claim 9 or 10, wherein the tapered end (100A; 100A'; 100A'; 100A*; 100A**; 100A***) of the elongated rod member (100; 100*) is further delineated by a pair of side surfaces (SC; SC*) forming side bevels alongside at least a portion of the bevelled surface (SA; SA*) and of the recessed surface (SB; SB*),
and wherein an angle of inclination (δ; δ*) of each side surface (SC; SC*) with respect to the recessed surface (SB; SB*) is preferably comprised between 25° and 35°.

12. The medical instrument (10; 10*) according to any one of claims 9 to 11, wherein an angle of inclination (γ; γ*) of a plane comprising the bevelled surface (SA; SA*) with respect to the defined plane (P0) is comprised between 10° and 15°.

13. The medical instrument (100; 110) according to any one of claims 9 to 12, wherein a thickness (t; t+h; t*; t*+h*) of the tapered end (100A; 100A'; 100A"; 100A*; 100A**; 100A***), as measured perpendicularly to the defined plane (P0),does not exceed 1 mm, and/or
wherein a width (w; w*) of the tapered end (100A; 100A'; 100A"; 100A*; 100A**; 100A***), as measured within the defined plane (P0),does not exceed 2 mm.

14. The medical instrument (100; 110) according to any one of claims 9 to 13, wherein the tapered end (100A'; 100A"; 100A**; 100A***) includes a longitudinal ridge (150'; 150"; 150**; 150***) that projects away from the recessed surface (SB; SB*),
wherein a height (h; h*) of the longitudinal ridge (150'; 150"; 150**; 150***), as measured perpendicularly to the defined plane (P0),preferably does not exceed 0.5 mm;
and wherein a leading edge of the longitudinal ridge (150'; 150"; 150**; 150***) is optionally tapered and stops short of a leading edge of the tapered end (100A'; 100A"; 100A**; 100A***).

15. The medical instrument (10; 10*) according to any one of the preceding claims, wherein the elongated rod member (100; 100*) extends along a generatrix (GX) and wherein a lateral breadth of the elongated rod member (100; 100*) as measured at any point along the generatrix (GX), up to an including the distal end of the elongated rod member (100; 100*), does not exceed 2 mm, and/or
wherein a cross-sectional area of the elongated rod member (100; 100*) does not exceed 2.5 mm², and/or
wherein the elongated rod member (100; 100*) has a substantially circular or polygonal cross-section, upstream of the distal end of the elongated rod member (100; 100*), whose lateral breadth does not exceed 1.5 mm.
